Europäisches Patentamt

(19) European Patent Office

Office européen des brevets

(11) Publication number: **0 310 540**
**A2**

(12) **EUROPEAN PATENT APPLICATION**

(21) Application number: 88730220.6

(22) Date of filing: 30.09.88

(51) Int. Cl.⁴: **C 07 D 409/12**
**A 01 N 43/40**

(30) Priority: 02.10.87 DE 3733806

(43) Date of publication of application:
05.04.89 Bulletin 89/14

(84) Designated Contracting States:
AT BE CH DE ES FR GB GR IT LI LU NL SE

(71) Applicant: SCHERING AKTIENGESELLSCHAFT Berlin
und Bergkamen
Müllerstrasse 170/178 Postfach 65 03 11
D-1000 Berlin 65 (DE)

(72) Inventor: Hübl, Dieter, Dr.
Massmannstrasse 9
D-1000 Berlin 41 (DE)

Pieroh, Ernst-Albrecht
Im Fischgrund 18
D-1000 Berlin 28 (DE)

(54) 2-Imino-1,3-dithietanes and compositions for combating nematodes.

㊗ The invention relates to the use of 2-(2-pyridy-limino)-1,3-dithietane or an acid addition salt thereof for combating nematodes and also to novel acid addition salts of 2-(2-pyridylimino)-1,3-dithietane.

EP 0 310 540 A2

**Description**

## 2-IMINO-1,3-DITHIETANES AND COMPOSITIONS FOR COMBATING NEMATODES

The invention relates to new acid addition salts of 2-(2-pyridylimino)-1,3-dithietane and nematicidal compositions containing 2-(2-pyridylimino)-1,3-dithietane and its acid addition salts.

2-(2-Pyridylimino)-1,3-dithietane is a known compound witb insecticidal and acaricidal activity (US 3,975,528). Besides this, 2-imino-1,3-dithietanes with the same activity are known (US 3,928,382, US 3,997,668 and US 4,009,279).

The object of the present invention is to provide active compounds and compositions for combating nematodes. which improve the state of the art.

surprisingly it has now been found that It has now been found that 2-(2-pyridylimino)-1,3-dithietane and its acid addition salts have good nematicidal activity along with good plant compatibilty.

The acid addition salts of the compounds of the invention of formula I can be derived from inorganic and organic acids. Examples are hydrochloric acid, hydrobromic acid, hydroiodic acid, sulphuric acid, mono and difunctional carboxylic acids and hydroxycarboxylic acids, such as for example acetic acid, trifluoroacetic acid, maleic acid, fumaric acid and citric acid, as well as sulphonic acids, such as for example p-toluenesulphonic acid, 1,5-naphthalenedisulphonic acid and trifluoromethanesulphonic acid.

The acid addition salts can be obtained in conventional manner, for example by dissolving the 2-(2-pyridylimino)-1,3-dithietane in a suitable solvent and adding the corresponding acid.

Because of the nematicidal activity coupled with good piant compabiblity, the compound according to the invention can be successfully applied in plant protection as pesticides in agriculture, in vine and fruit growing, in horticulture and in forestry.

Plant parasitic nematodes which can be controlled according to the invention include for example root-knot nematodes, such as Meloidogyne incognita, Meloidogyne hapla and Meloidogyne javanica, cyst forming nematodes, such as Globodera rostochiensis, Heterodera schacktii, Heterodera avanae, Heterodera glycines and Heterodera trifolii, and stem and leaf eelworms, such as Ditylenchus dipsaci, Ditylenchus destructor, Aphelenchoides ritzemabosi, Pratylenchus neglectus, Pratylenchus penetrans, Pratylenchus curvitatus, as well as Tylenchorhynchus dudius, Tylenchorhynchus claytoni, Rotylenchus robustus, Heliocotylenchus multicinctus, Radopholus similis, Belonolaimus longicaudatus, Longidorus elongatus and Trichodorus primitivus.

The active ingredients of the invention can be used in the form of their commercial formulations and/or in the form of preparations obtained from these formulations.

The content of active ingredient used in the preparations prepared from the commercial formulations can vary over wide ranges. The rate of use of combating per hectare lies between about 0.03 kg to about 10 kg, preferably about 0.3 kg to about 6 kg.

The active ingredient can be applied in the usual formulations such as solutions, emulsions, wettable powders, suspensions, powders, dusts, foams, pastes, soluble powders, granules, aerosols, suspension concentrates, seed dressings, natural and synthetic substances impregnated with the active ingredients, microcapsules in polymers and in seed coatings for seeds, as well as formulations with burning substances such as smoke cartridges, smoke capsules and smoke spirals amongst others as well as ULV-cold and hot fogging formulations.

These formulations can be prepared in known manner for example by mixing the active ingredient with diluents such as liquid solvents, and liquefied gases and/or solid carriers, optionally using surface active aqents such as emulsifiers and/or dispersing agents and/or foaming agents.

When using water as the diluent, organic solvents can also be used for example as auxiliary solvents.

Examples of liquid solvents include aromatic hydrocarbons, such as xylene, toluene or alkynaphthalene, chlorinated aromatic or chlorinated aliphatic hydrocarbons, such as chlorobenzene, chloroethylene or methylene dichloride, aliphatic hydrocarbons, such as cychlohexane or paraffins, for example mineral oil fractions, alcohols, such as butanol and glycol as well as their ethers and esters, ketones, such as acetone, methyl ethyl ketone, methyl isobutyl ketone or cyclohexanone, strongly polar solvents, such as dimethylformamide and dimethyl sulphoxide, as well as water.

By the term liquefied gaseous diluents or carriers are meant those substances which are gaseous at normal temperature and pressure, for example aerosol blowing agents, such as halohydrocarbons, as well as butane, propane, nitrogen and carbon dioxide.

Examples of solid carriers are natural earth powders, such as kaolin, alumina, talc, chalk, quartz, attapulgite, montmorillonite or diatomaceous earths and synthetic powders, such as finely divided silica, aluminium oxide and silicates as well as solid carriers for granules, crushed and fractionated natural rocks, such as calcite, marble, pumice, sepiolith and dolomite, as well as synthetic granules from inorganic and organic powders as well as granules from organic materials such as sawdust, coconut shells, maize cobs amd tobacco stalks.

Examples of emulsifying and/or foaming agents include non-ionic and anionic emulsifiers, such as polyoxyethylene-fatty acid esters, polyoxyethylene-fatty alcohol ethers, for example alkylaryl-polyglycol-ethers, alkylsulphonates and arylsulphonates as well as protein hydrolysates.

Dispersing agents include for example lignin, sulphite waste liquors and methylcellulose.

There can also be used in the formulations sticking agents such as carboxymethylcellulose, natural and synthetic powdery, granulated or latex-forming polymers, as well as gum arabic, polyvinyl alcohol and polyvinyl acetate.

There can also be used dyestuffs, such as inorganic pigments, for example iron oxide, titanium oxide, ferrocyan blue and organic dyestuffs such as alizarin- and azo-metal phthalocyanine dyestuffs and trace elements such as salts of iron, manganese, boron, copper, cobalt, molybdenum and zinc.

The formulations contain in general between 0.1 and 95 weight percent of 2-(2-pyridylimino)-1,3-dithietane, preferably between 0.5 and 90 percent.

The following examples illustrate the preparation of compounds according to the invention.

<u>Example 1</u>

2-(2-Pyridylimino)-1,3-dithietane, hydrochloride

3.50 g (0.019 mol) 2-(2-Pyridylimino)-1,3-dithietane was dissolved in 300 ml diethyl ether and, under vigorous stirring and ice cooling, hydrogen chloride gas was passed in. The precipitated crystals were separated and dried.

Yield: 3.40 g = 80.9% of theory
m.p.: 157°C

In a similar manner the following acid addition salts were prepared:

| Example No | HX | | | m.p.(°C) |
|---|---|---|---|---|
| 2 | | | $H_2SO_4$ | 136-138 |
| 3 | 1/2 | x | (naphthalene-1,5-disulfonic acid, $SO_3H$ / $SO_3H$) | 210 |
| 4 | | | HBr | 192 |
| 5 | | | HI | 155 |
| 6 | | | $HNO_3$ | 128 |
| 7 | 2 | x | $H_3PO_4$ | 107-109 |
| 8 | | | $H_2SO_3$ | |
| 9 | | | $CH_3COOH$ | |
| 10 | | | $(COOH)_2$ | |
| 11 | 1/2 | x | $HO_2C-CH_2-CO_2H$ | |
| 12 | | | $ClF_2C-COOH$ | |
| 13 | | | $ClCH_2-COOH$ | |
| 14 | | | $CF_3-COOH$ | |
| 15 | | | $CH_3-(CH_2)_3-COOH$ | |
| 16 | | | ($H_3C$-benzene-$SO_3H$) | |
| 17 | | | $HOCH_2-COOH$ | |
| 18 | 1/2 | x | $HO_2C-CH=CH-CO_2H$ | |

4

Examples of formulations are:

1. Wettable powder
10 parts by weight of 2-(2-pyridylimino)-1,3-dithietane are intimately mixed with 12 parts by weight of calcium lignosulphonate, 76 parts by weight of finely divided kaolin and 2 parts by weight of dialkylnaphthalene sulphonate and then milled.

II. Dusting powder
2,5 parts by weight of 2-(2-pyridylimino)-1,3-dithietane were dissolved in 10 methylene chloride and added to a mixture of 20 parts by weight of finely divided silicic acid and 71.5 parts by weight talc and 1 part by weight Sudan red. The solvent was removed in vacuo and the residue finely milled.

III. Granule
5 Parts by weight of 2-(2-pyridylimino)-1,3-dithietane were dissolved in 10 parts by weight of methylene chloride and sprayed onto 95 parts by weight granulated attapulgite of particle size 0.3 mm - 0.8 mm and dried.

IV. Emulsifiable concentrate
20 parts by weight of 2-(2-pyridylimino)-1,3-dithietane were dissolved in a mixture of 75 parts by weight of isophorone and 5 parts by weight of a mixture of 30 parts by weight of calcium benzene sulphonate and 30 parts by weight of castor oil polyglycolate with 40 mole % ethylene oxide and 40 parts by weight of a copolymer of propylene- and ethylene oxide.

Use Example 1

Control of root knot nematode (Meloidogyne incognita)
The composition of the invention was mixed thoroughly in the form of a 10% powder preparation with soil that had been strongly infested with the test nematode. After this the treated soil was put into a 0.5 litre fermenting tube, treated with cucumber seeds and cultivated at a soil temperature of 25-27°C in a greenhouse. After a cultivation time of 25 to 28 days, the cucumber roots were washed and inspected in a water bath for nematode attack (root knots) and the % level of activity of the active ingredients compared with a treated control was determined. When the nematode attack is fully controlled the level of activity is 100%.
At a dose of 10 mg of active substance per litre of soil, a nematode attack by Meloidogyne incognita was fully controlled (100%) by 2-(2-Pyridylimino)-1,3-dithietane and the acid addition salts of Examples 1-3.

Use Example 2

Ovicidal and/or inhibition of hatching activity on egg masses of root knot nematode (Meloidogyne incognita) and cysts of the root cyst nematode (Heterodera schachtii)
Egg masses of Meloidogyne incognita and ready to hatch cysts of Heterodera schachtii were immersed for 48 hours in 0.001% suspensions or emulsions of compounds of the invention washed in running water and left at 25-26°C until larva emergence.
The composition of the invention based on 2-(2-pyridylimino)-1,3-dithietane and its acid addition salts of of Examples 1-3 fully inhibited (100% activity) the hatch of $L_2$ larvae of Meloidgyne incognita and of Heterodera schachtii.

**Claims**

1. A method of combating nematodes which comprises applying to the nematode or its locus, a nematicidally effective amount of 2-(2-pyridylimino)-1,3-dithietene or an acid addition salt thereof.
2. A nematicidal composition which comprises 2-(2-pyridylimino)-1,3-dithietane or an acid addition salt thereof, in admixture with an agriculturally acceptable diluent or carrier.
3. A composition according to claim 2, which contains from 0.1 to 95% by weight of 2-(2-pyridylimino)-1,3-dithietane or acid addition salt thereof.
4. A composition according to claim 3, which contains from 0.5 to 90% by weight of 2-(2-pyridylimino)-1,3-dithietane or acid addition salt thereof.
5. An acid addition salt of 2-(2-pyridylimino)-1,3-dithietane.